# EUROPEAN PATENT APPLICATION

(11) **EP 2 292 265 A1**
(43) Date of publication of application: **09.03.2011**
(21) Application number: 09745859.0
(22) Date of filing: 05.05.2009
(51) Int. Cl.: A61K 39/395, A61K 31/7088, C07K 14/515

(54) **SET OF ANTIANGIOGENIC MOLECULES AND USE THEREOF**

(30) Priority: 06.05.2008 ES 200801291
(71) Applicant: Benet Ferrus, David, 08192 Sant Quirze del Vallés (ES); De Borja Corcostegui, Francisco, 08192 Sant Quirze del Vallés (ES); Prieto García, José Alberto, 08192 Sant Quirze del Vallés (ES)
(72) Inventor: Benet Ferrus, David, 08192 Sant Quirze del Vallés (ES); De Borja Corcostegui, Francisco, 08192 Sant Quirze del Vallés (ES); Prieto García, José Alberto, 08192 Sant Quirze del Vallés (ES)
(74) Representative: Isern-Jara, Nuria
(86) International application number: PCT/ES2009/000236
(87) International publication number: WO 2009/138533

(57) **Abstract**

The present invention relates to an antiangiogenic pharmaceutical composition comprising at least one molecule selected from the group consisting of an antisense nucleic acid molecule of VEGF 165, an antisense nucleic acid molecule of alpha-9 domain that forms integrin alpha-9/beta-1 and an antisense nucleic acid molecule of VAP-1, a molecule of VEGF 165-specific antibody, a molecule of antibody specific for the alpha-9 domain that forms integrin alpha-9/beta-1 and a molecule of VAP-1-specific antibody and/or combinations thereof and a pharmaceutically acceptable vehicle. The present invention also protects the use of said molecules for the production of a medicament.

## Description

### FIELD OF THE INVENTION

The present invention is comprised in the field of vascular disease therapy, particularly it relates to a group of molecules with antiangiogenic activity and to the use thereof in vascular diseases, more particularly to their use in cancer and/or eye diseases. The invention also relates to pharmaceutical compositions comprising said set of molecules with antioangiogenic activity and to the use of said compositions in the production of medicaments useful in anticancer and/or eye therapy.

### BACKGROUND OF THE INVENTION

Angiogenesis is a physiological process consisting of the formation of new blood vessels from preexisting vessels. Angiogenesis is a normal phenomenon during embryonic development, body growth and wound healing. However, it is also a primary process during malignant transformation and tumor growth.

For angiogenesis to occur, endotelial vascular cells must invade surrounding tissue and proliferate on the apex of a new capillary vessel. Both processes, invasion and proliferation, are repeated sequentially until the new capillary network is completely established. Initially, certain endotelial cells degrade their basal membrane and form very small buds that permeate the perivascular connective tissue. These buds are formed by migration of endotelial cells to the apex and through migration of other endotelial cells below the apex level. As buds elongate, a lumen is gradually formed on the inside. In this way, hollow tubes are formed, which anastomose to each other, forming capillary loops through which blood may start circulating. From the newly formed capillaries new buds can emerge, eventually giving rise to a complete capillary network *[*Hanahan D., Weinberg R.A. The hallmarks of cancer. Cell 100:57-70, 2000*;* Hobson B., Denekamp J. Endothelial proliferation in tumours and normal tissue: continuous labelling studies. Br. J. Cancer 49:405-13, 1984*;* Hanahan D., Folkman J. Patterns and emerging mechanisms of the angiogenic switch during tumorigenesis. Cell 86:353-64, 1996*;* Carmeliet P. Mechanisms of angiogenesis and arteriogenesis. Nat. Med. 6:389-95, 2000*].*

The blood capillaries comprise endotelial cells and pericytes. These two cell types are sufficient to complete the formation of the capillary network. Specific angiogenic molecules can initiate this process, which can be halted by specific inhibitors. These molecules, which have opposing functions, act continuously and in a coordinated manner in one direction or in the opposite direction, depending on the physiologic requirements that are more important at a given time. Thus, in the same way they maintain the vascular network in a quiescent state, they can also cause a rapid proliferation of the vascular endotelial cells, as occurs, for example, during wound healing. Whether the angiogenesis process occurs or not is the outcome of a fine balance between positive and negative signals.

Angiogenesis is involved in many physiological processes, including wound healing, bone fracture repair, the menstrual cycle, etc. On the other hand, many pathologies are dependent on angiogenesis, such as rheumatic arthritis, psoriasis, bartonelosis, transplanted organ rejection, hemorrhages and ocular neovascularization (one of the most frequent causes of blindness), such as, for example, age-related macular degeneration, diabetic retinopathy, macular edema and uveitis. Also, angiogenesis plays an important role in the progressive growth and metastatic dispersion of tumors. A tumor needs to stimulate continuously the growth of new capillaries in order to grow. In addition, new vessels within the tumor provide malignant cells with a path through which they may enter circulation and establish metastasis in distant sites *[*Hanahan D., Weinberg R.A. The hallmarks of cancer. Cell 100:57-70, 2000*;* Hobson B., Denekamp J. Endothelial proliferation in tumours and normal tissue: continuous labelling studies. Br. J. Cancer 49:405-13, 1984*;* Hanahan D., Folkman J. Patterns and emerging mechanisms of the angiogenic switch during tumorigenesis. Cell 86:353-64, 1996*;* Carmeliet P. Mechanisms of angiogenesis and arteriogenesis. Nat. Med. 6:389-95, 2000*;* Maltepe E., Simon M.C. The role of HIF-1 and ARNT proteins in blood vessel development. In: Angiogenesis in health and disease (Rubany G.M., Ed.), Marcel Dekker, New York, pp 133-144*;* Kaban K., Herbst R. S. Angiogenesis as a target for cancer therapy. Hematol. Oncol. Clin. North Am. 16:1125-71, 2002*;* Rakic J.M., Maillard C., Jost M., Bajou K., Masson V., Devy L., Lambert V., Foidart J.M., Noel A. Role of plasminogen activator-plasmin system in tumor angiogenesis. Cell Mol. Life Sci. 60:463-73, 2003*;* Bazzoni G., Dejana E., Lampugnani M.G. Endotelial adhesion molecules in the development of the vascular tree. Curr. Opin. Cell Biol. 11:573-81, 1999*;* Lafleur M.A., Forsyth P.A., Atkinson S.J., Murphy G., Edwards D.R. Perivascular cells regulate endothelial membrane type-1 matrix metalloproteinase activity. Biochem. Biophys. Res. Commun. 282:463-73, 2001*;* Folkman J. Role of angiogenesis in tumor growth and metastasis. Semin. Oncol. 29(suppl.16):15-8, 2002*].*

Significant progress has been made in recent years especially in the field of ocular pathologies, which has allowed to develop specific inhibitors for the angiogenic factors present in the eye, such as VEGF 165 (*Vascular Endothelial Growth Factor*), which is the factor involved in ocular neovascularization already described a few years ago *[*Napoleone Ferrara & Robert S. Kerbel, Angiogenesis as a therapeutic target, Nature 438:967-971, 15 December 2005*].* This factor needs cofactors for adequate activation of the production of new vessels, requiring previous steps for their synthesis and for the production of said factor. Among said cofactors are the so-called adhesion molecules, which play an important role in the translation of proliferation and differentiation signals of new vessels, including the so-called integrins, which are heterodynamic membrane proteins that can mediate in cell adhesion, migration and proliferation. Indirect evidence suggests that the inhibition of integrin alpha-3/beta-1 inhibits VEGF. Likewise, it has also been described that integrins alpha-5/beta-5, alpha-5/beta-1 and alpha-4/beta-1 are involved in angiogenesis. For its part, the vascular adhesion protein 1, VAP-1, also called SSAO (*Human Semicarbazide-Sensitive Amine Oxidase*) is an amine oxidase that contains copper and that has both enzymatic and adhesive functions. VAP-1 catalizes the oxidative deamination of primary amines, giving rise to the formation of the respective aldehyde and to the release of hydrogen peroxide and ammonia. Membrane-linked VAP-1 is an inflammation-inducible endothelial cell-adhesion molecule that mediates the interaction between white cells and activated endotelial cells in inflamed vessels. Both a direct adhesive function and an enzymatic function seem to be involved in the adhesion cascade.

The idea of using nucleic acid molecules as therapeutic agents was conceived a few years ago, as antisense strategies were developed. Antisense compounds are single-chain nucleic acids that, in principle, block the synthesis of the target protein through sequence-dependent hybridization to the encoding mRNA. The mechanism of action based on nucleic acid aptamers is completely different. Aptamers are single-chain nucleic acids that directly inhibit the protein function through their folding into a specific three-dimensional structure that determines de high-affinity linkage to the target protein.

Considering all state-of-the-art knowledge published to date, it can be appreciated that the use of monoclonal antibodies individually only inhibits circulating VEGF 165. Therefore, the present inventors have determined that the most adequate way for blocking the largest amount of VEGF 165 is by use of a pharmaceutical composition comprising several factors causing angiogenesis, in this case the combination of different antibodies (anti-VEGF, anti-VAP1 and anti-Alpha9) and anti-siRNAs of said factors (siRNA-anti-VEGF, siRNA-anti-VAP1 and siRNA-anti-Alpha9), and this aspect is not obvious for an expert on the basis of the state-of-the-art.

According to all what has been disclosed above, it must be noted that, although vascular problems have been extensively studied for many years, the need still exists for finding therapeutic alternatives for the treatment of vascular diseases derived from angiogenic processes. Therefore, the need still exists today for finding molecules with a antiangiogenic capacity that are capable of remitting vascular alterations that lead to diseases such as cancer and/or ocular disease, providing an efficacious remedy as an alternative to those treatments already described in the state-of-the-art.

### DETAILED DESCRIPTION OF THE INVENTION

The object of the present invention is an antiangiogenic pharmaceutical composition comprising a pharmaceutically acceptable amount of at least one molecule selected from the group consisting of an antisense nucleic acid molecule of VEGF 165, an antisense nucleic acid molecule of alpha-9 domain that forms integrin alpha-9/beta-1, and an antisense nucleic acid molecule of VAP-1, a molecule of VEGF 165-specific antibody, a molecule of antibody specific for the alpha-9 domain that forms integrin alpha-9/beta-1, and a molecule of VAP-1-specific antibody and/or combinations thereof and a pharmaceutically acceptable vehicle.

According to a preferred embodiment of the invention, the composition comprises at least two molecules selected from the group consisting of an antisense nucleic acid molecule of VEGF 165, an antisense nucleic acid molecule of the alpha-9 domain that forms integrin alpha-9/beta-1, and an antisense nucleic acid molecule of VAP-1.

According to another preferred embodiment of the invention, the composition comprises an antisense nucleic acid molecule of VEGF 165 and an antisense nucleic acid molecule of alpha-9 domain that forms integrin alpha-9/beta-1.

According to another preferred embodiment of the invention, the composition comprises an antisense nucleic acid molecule of VEGF 165 and an antisense nucleic acid molecule of VAP-1.

According to another preferred embodiment of the invention, the composition comprises an antisense nucleic acid molecule of the alpha-9 domain that forms integrin alpha-9/beta-1 and an antisense nucleic acid molecule of VAP-1.

According to another preferred embodiment of the invention, the composition comprises an antisense nucleic acid molecule of VEGF 165, an antisense nucleic acid molecule of the alpha-9 domain that forms integrin alpha-9/beta-1, and an antisense nucleic acid molecule of VAP-1.

According to another preferred embodiment of the invention, the composition comprises at least two molecules selected from the group consisting of an antibody molecule specific for VEGF 165, an antibody molecule specific for the alpha-9 domain that forms integrin alpha-9/beta-1, and an antibody molecule specific for VAP-1.

According to another preferred embodiment of the invention, the composition comprises an antibody molecule specific for VEGF 165 and an antibody molecule specific for the alpha-9 domain that forms integrin alpha-9/beta-1.

According to another preferred embodiment of the invention, the composition comprises an antibody molecule specific for VEGF 165 and an antibody molecule specific for VAP-1.

According to another preferred embodiment of the invention, the composition comprises an antibody molecule specific for the alpha-9 domain that forms integrin alpha-9/beta-1, and an antibody molecule specific for VAP-1.

According to another preferred embodiment of the invention, the composition comprises an antibody molecule specific for VEGF 165, an antibody molecule specific for the alpha-9 domain that forms integrin alpha-9/beta-1, and an antibody molecule specific for VAP-1.

Another object of the present invention is the use of the above mentioned pharmaceutical composition for the manufacturing of a medicament for the treatment of a pathological process in which blood vessel proliferation takes place. More specifically, for the production of a medicament for the treatment of a pathological process in which the blood vessel proliferation is age-related macular degeneration, diabetic retinopathy, macular edema, uveitis or cancer.

Another object of the present invention is the use of the pharmaceutical composition for the manufacturing of a medicament for the treatment of retinal neovascularization having a prolongued therapeutical effect, and the use of said pharmaceutical composition by the intravitreal route.

Another object of the present invention is a treatment method of a pathological process in which proliferation of blood vessels takes place, consisting of administering to a patient the above mentioned pharmaceutical composition.

The molecules of the present invention are useful as modulating agents of the regulation and production of new vessels, which are present in the above described diseases. Thus, with the antisense nucleic acid molecules, the production of new vessels is blocked through the blocking of the messenger RNA of the above described factors, and with the specific monoclonal antibody molecules, said circulating factors are blocked.

These molecules can equally be used in combination or individually. Thus, a patient can be treated with a combination of the antisense nucleic acid molecules (siRNA-anti-VEGF, siRNA-anti-VAP1 and siRNA-anti-ALPHA9) to block the production of new vessels through the blocking of the messenger RNA of the above described factors, either with a combination of the specific monoclonal antibody molecules (Anti-VEGF, Anti-VAP1 and Anti-ALPHA9) blocking said circulating factors in the bloodstream, or with a combination of the antisense nucleic acid molecules and monoclonal antibody molecules (siRNA-anti-VEGF, siRNA-anti-VAP1, siRNA-anti-ALPHA9, Anti-VEGF, Anti-VAP1 and Anti-ALPHA9).

The antisense nucleic acid molecules of the present invention can be obtained by classical genetic engineering techniques known of the state-of-the-art.

For their part, the specific monoclonal antibody molecules of the present invention can be obtained from hybridoma cell lines, conjugating the peptides of the typical proteins, fusing and expanding the cell lines and subsequently subcloning them.

### EMBODIMENT EXAMPLES

The specific examples provided hereinbelow are illustrative of the nature of the present invention. These examples are included with illustrative purposes only and should not be construed as limiting of the invention herein claimed.

### Example 1: blockage of the expression of angiogenic factors with antisense nucleic acid molecules

To carry out the blockage of the expression of the angiogenic factors by use of the set of antisense nucleic acid molecules, an effective amount of said set is administered to the subject and the degree of inhibition of the expression is evaluated by using techniques for determining nucleic acids.

### Example 2: blockage of circulating angiogenic factors with specific monoclonal antibody molecules

To carry out the blockage of the circulating angiogenic factors by use of the set of specific monoclonal antibody molecules, an effective amount of said set is administered and circulating levels of said factors are determined by using techniques for determining proteins.

### Example 3: evaluation of toxicity for the retina

The purpose of this study was to evaluate the possible retinal toxicity effect caused by three treatments administered separately and in combination (treatments 1, 2 and 3 of Table 1) by the intravitreal route in white New Zealand (NZW) rabbits.

The three treatments tested are combinations of different monoclonal antibodies with synthetic peptides and synthetic interference RNAs (siRNA), including a transporter. For the present example, the names used in tables are those for antigens, i.e.: Anti-VEGF, Anti-VAP1 and Anti-Alpha9.

**TABLE 1**

| TREATMENT | Antibody | siRNA | Transporter |
|---|---|---|---|
| 1 | Anti-VEGF | siRNA-anti-VEGF | SD 1205 |
| 2 | Anti-VAP1 | siRNA-anti-VAP1 | SD 1205 |
| 3 | Anti-ALPHA9 | siRNA-anti-ALPHA9 | SD 1205 |

All assays were carried out with a 0.75 µg dose of antibody in each eye and were administered by intravitreal route in a volume of 0.05 µl in each eye.

A preliminary oftalmoscopic study was made for each and all animals to confirm their viability for the study.

After the administration of the different antibodies, a series of oftalmologic studies was performed, on days 1, 5, 8, 12 y 33 post-administration.

After each oftalmologic examination, one animal from each group was sacrificed, its two eyes excised and introduced in 10% formalin, and stored in the cold.

The purpose of the study was to evaluate the potential retinal toxicity effect caused by the three combinations (treatments 1, 2 and 3 of Table 1) administered individually and in combination by intravitreal route in NZW rabbits.

The purpose of administering the combination of substances was to obtain a treatment for the retinal neovascularization having a more prolonged therapeutical effect.

The intravitreal route was selected because it is the route intended for use with humans.

### Administration route and dose:

The treatments were administered by the intravitreal route in an amount of 50 ml of solution in the right eye (this route was used because it is the one anticipated to be used in humans).

The dose administered in each of the assays was 0.75 µg/eye. The control group received, by the same route in the right eye, 50 µl of vehicle (PBS in water for injectables), which was the same vehicle used to prepare the different treatment samples.

### Preparation of the compositions:

The concentrations of antibody in each of the compositions were the following (see Table 2):

**TABLE 2**

| TREATMENT | Antibody | Concentration of antibody |
|---|---|---|
| 1 | Anti-VEGF | ∼1.5 mg/ml |
| 2 | Anti-VAP1 | ∼0.75 mg/ml |
| 3 | Anti-ALPHA9 | ∼1.5 mg/ml |

According to the concentration of antibody, a solution of 15 µg/ml in PBS was prepared and the amount of each of the final combinations was obtained by mixing both substances (antibody + siRNA). In this way, the final solutions in a volume of 10 ml obtained were the following (see Table 3):
each animal was administered a dose of 0.75 µg of antibody in each eye, giving a final volume of 0.05 µl/eye.

**TABLE 3**

| Final solution (10 ml final volume) | | |
|---|---|---|
| TREATMENT | Concentration of antibody | Concentration of siRNA + Transporter |
| 1 | 0.05 ml Anti-VEGF | 0.05 ml siRNA-anti-VEGF + SD 1205 |
| 2 | 0.1 ml Anti-VAP1 | 0.1 ml siRNA-anti-VAP1 + SD 1205 |
| 3 | 0.05 ml Anti-ALPHA9 | 0.05 ml siRNA-anti-ALPHA9 + SD 1205 |

| COMBINATION: | | |
|---|---|---|
| 4 | 0.05 ml Anti-VEGF + 0.1 ml Anti-VAP1 + 0.05 ml Anti- ALPHA9 | 0.05 ml siRNA-anti-VEGF + SD 1205 + 0.1 ml siRNA-anti-VAP1 + SD 1205 + 0.05 ml siRNA-anti-ALPHA9 + SD 1205 |

### Experimental design:

Before the administration of each of the treatments by intravitreal route to all animals, a preliminary oftalmoscopy study was done to confirm the viability of the study. This study was performed using a high-resolution microscope after inducing midriasis with the topical eye administration of 2.5% phenylephrine with 0.5% tropicamide. The animals were anesthesized with a combination of ketamine hydrochloride (50 mg/kg) and xilacine hydrochloride (5 mg/kg) together with an anesthetic topic combination containing tetracaine (0.1%) and oxybuprocaine (0.4%).

The treatments were administered by intravitreal route in 50 ml of solution in the right eye (this route was used because it is the one anticipated to be used in humans).

The dose administered in each of the assays was 0.75 µg/eye. The control group received, through the same route in the right eye, 50 µl of vehicle (PBS in water for injectables), which is the same vehicle used to prepare the different treatment samples.

After administering the different treatments, different oftalmologic studies were conducted on days 1, 5, 8, 12 and 33 post-administration.

After each of the oftalmologic examinations, one animal from each group was sacrificed with an overdose of sodium phenobarbital, both eyes from each animal were excised and introduced into 10% formaline and stored in the cold. Once in the formaline solution, a punction was performed immediately under the iris with a 30G needle to allow formolization of the inside.

No anomalous values were observed compared to the weight of the animals during the study. In addition, no alteration was observed in the behaviour or state of health of the animals during the study period, apart from the alterations observed during the oftalmologic evaluation.

The purpose of administering the combination of substances was to obtain a treatment for the retinal neovascularization having a more prolonged therapeutical effect.

The results obtained demonstrate that neither the administration of each antibody with the respective siRNA + Transporter, nor of the combinations thereof, gave rise to retinal toxicity, and can therefore be used individually or in combination for the treatment of diseases.

## Claims

1. Antiangiogenic pharmaceutical composition **characterized in that** it comprises a pharmaceutically acceptable amount of at least one molecule selected from the group consisting of an antisense nucleic acid molecule of VEGF 165, an antisense nucleic acid molecule of the alpha-9 domain that forms integrin alpha-9/beta-1 and an antisense nucleic acid molecule of VAP-1, an antibody molecule specific for VEGF 165, an antibody molecule specific for the alpha-9 domain that forms integrin alpha-9/beta-1 and an antibody molecule specific for VAP-1 and/or combinations thereof and a pharmaceutically acceptable vehicle.

2. Use of the pharmaceutical composition of claim 1, for the production of a medicament for the treatment of a pathological process in which blood vessel proliferation takes place.

3. Use of the pharmaceutical composition according to claim 2, for the production of a medicament for the treatment of a pathological process in which the blood vessel proliferation is age-related macular degeneration, diabetic retinopathy, macular edema, uveitis or cancer.

4. Use of the pharmaceutical composition according to any of claims 2 and 3, for the production of a medicament for the treatment of retinal neovascularization having a prolonged therapeutic effect.

5. Use of the pharmaceutical composition of claim 1 by the intravitreal route.

6. Method of treatment of a pathological process in which blood vessel proliferation takes place, consisting of the administration in a patient of the pharmaceutical composition of claim 1.
